# EUROPÄISCHE PATENTSCHRIFT

(11) **EP 1 630 153 B1**
(45) Veröffentlichungstag und Bekanntmachung des Hinweises auf die Patenterteilung: **12.12.2007**
(21) Anmeldenummer: 05015396.4
(22) Anmeldetag: 15.07.2005
(51) Int. Cl.: C07C 67/08, C07C 69/54

(54) **Verfahren zur Herstellung von (Meth)acrylsäureestern**
Process for the production of (meth)acrylic acid esters
Procédé de préparation d'esters d'acide (meth)acrylique

(30) Priorität: 04.08.2004 DE 102004038013; 11.05.2005 US 679668 P
(43) Veröffentlichungstag der Anmeldung: 01.03.2006
(73) Patentinhaber: BASF Aktiengesellschaft, 67056 Ludwigshafen (DE)
(72) Erfinder: Schröder, Jürgen, Dr., 67071 Ludwigshafen (DE)

(56) Entgegenhaltungen:
- EP-A- 0 202 610
- EP-A- 0 304 757

## Beschreibung

Die vorliegende Erfindung betrifft ein Verfahren zur Inhibierung der Polymerisation bei der Herstellung von (Meth)acrylsäureestern von C₁-C₈-Alkoholen.

Der Begriff (Meth)acrylsäure steht in dieser Schrift verkürzend für Methacrylsäure und/oder Acrylsäure, (Meth)acrylsäureester für Methacrylsäureester und/oder Acrylsäureester.

Die auf Basis von (Meth)acrylsäureestern hergestellten Polymere beziehungsweise Copolymere sind in Form von Polymerdispersionen von großer wirtschaftlicher Bedeutung. Sie finden beispielsweise Anwendung als Klebstoffe, Anstrichmittel oder Textil-, Leder- und Papierhilfsmittel.

(Meth)acrylsäure und (Meth)acrylsäureester sind polymerisationsfähige Verbindungen, daher ist in allen Verfahrensschritten auf eine ausreichende Polymerisationsinhibierung zu achten.

Die Herstellung von (Meth)acrylsäureestern ist vielfach beschrieben, siehe beispielsweise Kirk-Othmer, Encyclopedia of Chemical Technology, 4. Auflage, Band 1, Seiten 301 und 302., John Wiley & Sons, 1991.

In DE 198 51 983 A1 werden ebenso wie in EP 0 609 127 A1 Verfahren zur Herstellung von (Meth)acrylsäureestern beschrieben, bei denen das Reaktionswasser azeotrop aus dem Reaktionsgemisch abdestilliert wird. Der Rohester wird anschließend extraktiv gereinigt. In den wässrigen Phasen enthaltender Alkohol wird über eine Alkoholrückgewinnung in das Verfahren zurückgeführt.

DE 198 51 983 A1 empfiehlt zusätzlich die Rückspaltung der bei der Veresterung entstehenden Michael-Additions-Produkte und die Rückführung in die Veresterung.

In EP 0 609 127 A1 wird bei der Herstellung von Butylacrylat zusätzlich die Hydrolyse der in der wässrigen Phase der Extraktionsstufe enthaltenen Butylschwefelsäure zu Butanol und Schwefelsäure vorgeschlagen. Zur Vermeidung von Korrosion in der Butanolrückgewinnung wird die wässrige Phase nach der Hydrolyse neutralisiert.

In DE 196 04 252 A1 und EP 0 984 918 B1 werden ebenfalls Verfahren beschrieben, bei denen das Reaktionswasser azeotrop aus dem Reaktionsgemisch abdestilliert wird. Anschließend wird der Ester in einer weiteren Destillationskolonne durch azeotrope Destillation von nicht umgesetzter Acrylsäure und Hochsiedern, wie beispielsweise Veresterungskatalysatoren, Polymerisationsinhibitoren und Michael-Addukten, abgetrennt. Auch diese Verfahren verfügen über eine Alkoholrückgewinnung.

Des weiteren beschreiben US 6,172,258 und US 6,18,820 Verfahren, bei denen das Reaktionswasser gemeinsam mit Butylacrylat aus dem Reaktionsgemisch abdestilliert wird. Auch diese Verfahren verfügen über eine Butanolrückgewinnung.

Nachteilig bei den vorgenannten Verfahren ist, dass die abgetrennten wässrigen Phasen noch geringe Mengen an (Meth)acrylsäure und oder (Meth)acrylsäureester von C₁-C₈-Alkoholen enthalten. Durch unerwünschte Polymerisation entstehen daraus Ablagerungen. Diese Polymerisation lässt sich auch bei Zusatz von Polymerisationsinhibitoren nur unzureichend verhindern.

Die Inhibierung der Polymerisation durch Zusatz von Polymerisationsinhibitoren bei der Herstellung von (Meth)acrylsäureestern wird in EP 0 202 610 A2 offenbart.

Es bestand nun die Aufgabe, ein Verfahren zur Herstellung von (Meth)acrylsäureestern von C₁-C₈-Alkoholen zu finden, bei dem die Polymerisation in den wässrigen Phasen verhindert wird.

Gelöst wurde die Aufgabe durch ein Verfahren zur Inhibierung der Polymerisation bei der Herstellung von (Meth)acrylsäureestern von C₁-C₈-Alkoholen, in dem der pH-Wert des der C₁-C₈-Alkoholrückgewinnung zugeführten Reaktionswassers größer 7 ist.

Generell werden (Meth)acrylsäureester von C₁-C₈-Alkoholen durch direkte Veresterung von (Meth)acrylsäure mit C₁-C₈-Alkholen hergestellt. Das bei der Veresterung freigesetzte Wasser wird in dieser Schrift als Reaktionswasser bezeichnet.

Dabei umfassen die in der Literatur bekannten Verfahren mehrere Verfahrensschritte, die sich jedoch durch einige Varianten voneinander unterscheiden können.

Prinzipiell umfasst ein Verfahren zur Herstellung von (Meth)acrylsäureestern von C₁-C₈-Alkoholen folgende Schritte:
a) azeotrope Veresterung,
b) Abtrennung des Reaktionswassers und
c) Rückgewinnung des C₁-C₈-Alkohols aus dem Reaktionswasser.

Alternativ kann noch zwischen die Verfahrensschritte b) und c) die azeotrope Abtrennung des (Meth)acrylsäureesters von C₁-C₈-Alkohofen erfolgen, so dass das Verfahren folgende Schritte umfasst:
a) azeotrope Veresterung,
b) Abtrennung des Reaktionswassers,
c) azeotrope Abtrennung des (Meth)acrylsäureesters von C₁-C₈-Alkoholen und
d) Rückgewinnung des C₁-C₈-Alkohols aus dem Reaktionswasser.

Eine weitere Verfahrensvariante zur Herstellung von (Meth)acrylsäureestem von C₁-C₈-Alkoholen berücksichtigt die Rückspaltung der Michael-Additions-Produkte und die Rückführung der Spaltprodukte in die Veresterung, so dass diese Ausführungsform die folgenden Verfahrensschritte umfasst:
a) azeotrope Veresterung,
b) Abtrennung des Reaktionswassers,
c) Rückgewinnung des C₁-C₈-Alkohols aus dem Reaktionswasser,
d) Rückspaltung der Michael-Additions-Produkte aus dem Veresterungsrückstand und
e) Rückführung der Rückspaltungsprodukte in die Veresterung.

Alternativ dazu kann der (Meth)acrylsäureester von C₁-C₈-Alkoholen auch dadurch hergestellt werden, dass zusätzlich die azeotrope Abtrennung des Esters erfolgt:
a) azeotrope Veresterung,
b) Abtrennung des Reaktionswassers,
c) azeotrope Abtrennung des (Meth)acrylsäureesters von C₁-C₈-Alkoholen,
d) Rückgewinnung des C₁-C₈-Alkohols aus dem Reaktionswasser,
e) Rückspaltung der Michael-Additions-Produkte aus dem Veresterungsrückstand und
f) Rückführung der Rückspaltungsprodukte in die Veresterung.

Das erfindungsgmäße Verfahren zur Inhibierung der Polymerisation bei der azeotropen Veresterung von (Meth)acrylsäure mit C₁-C₈-Alkoholen nach einer der oben genannten Verfahrensvarianten zeichnet sich dadurch, dass der pH-Wert des der C₁-C₈-Alkoholrückgewinnung zugeführten Reaktionswassers größer 7 ist. Vorzugsweise ist der pH-Wert ≥ 7,5, bevorzugt ≥ 8, besonders bevorzugt ≥ 8,5 und insbesondere ≥ 9.

Der pH-Wert wird durch Zusatz prinzipiell aller üblichen alkalisch reagierenden Substanzen, bevorzugt Alkali- und Erdalkaliverbindungen, wie beispielsweise Natriumhydroxid, Kaliumhydroxid, Natriumcarbonat, Kaliumcarbonat, Natriumhydrogencarbonat, Kaliumhydrogencarbonat und/oder Calciumhydroxid eingestellt. Bevorzugt werden Natriumhydroxid, Natriumcarbonat und/oder Natriumhydrogencarbonat eingesetzt, besonders bevorzugt als wässrige Lösung. Dabei ist zu beachten, dass mit Hydrogencarbonaten als alleinige alkalisch reagierende Substanzen ein pH-Wert von ≥ 9 nicht erreicht werden kann. Die alkalisch reagierenden Substanzen werden deshalb auch als Gemische eingesetzt, können aber auch einzeln Anwendung finden.

Die alkalisch reagierenden Substanzen können an beliebiger Stelle dosiert werden, beispielsweise in den Phasenscheider am Kopf der Veresterungskolonne, in den Pufferbehälter vor der C₁-C₈-Alkoholrückgewinnung oder in eine beliebige Rohrleitung.

Wie im folgenden näher beschrieben werden bei der Herstellung von (Meth)acrylsäureestern von C₁-C₈-Alkoholen häufig Schwefelsäure und/oder Sulfonsäuren, wie beispielsweise p-Toluolsulfonsäure, Methansulfonsäure und/oder Dodecylbenzolsulfonsäure, eingesetzt. Dieselben Katalysatoren werden auch bei der Rückspaltung der Michaels-Additions-Produkte verwendet. Unter den gegebenen Reaktionsbedingungen, insbesondere bei den hohen Temperaturen der Rückspaltung, zersetzen sich die Katalysatoren teilweise und setzen Schwefeldioxid frei. Dieses Schwefeldioxid gelangt in das Reaktionswasser und kann dort nachgewiesen werden.

Die Rückspaltung der Michael-Additions-Produkte wird beispielsweise in EP 1 129 062 B1 und der dort zitierten Literatur beschrieben.

Vermutlich wird das freigesetzte Schwefeldioxid, das unter sauren und neutralen Bedingungen in Wasser als Redoxinitiator fungiert, im alkalischen Bereich als Redoxinitiator deaktiviert. Dadurch wird die Polymerisation wirksam verhindert, obwohl das Reaktionswasser noch Spuren von polymerisationsfähiger Verbindung enthält.

Da das Reaktionswasser nicht korrosiv ist, wurden bisher an die Reaktionswasser führenden Anlagenteile wie beispielsweise Leitungen oder Behälter keine besonderen Anforderungen gestellt. Üblicherweise wurde unlegierter Stahl verwendet. Aus dem unlegierten Stahl wurden jedoch geringe Mengen an Eisenionen herausgelöst.

In umfangreichen Versuchen wurde nun gefunden, dass Schwefeldioxid in Verbindung mit Metallionen, insbesondere Eisenionen, bereits in sehr geringen Mengen die Polymerisation von (Meth)acrylsäure/(Meth)acrylsäureester-Gemischen in sehr verdünnten wässrigen Lösungen induziert. Dies ist insbesondere dann ein Problem, wenn ein Pufferbehälter als Vorlage für die Rückgewinnung des C₁-C₈-Alkohols verwendet wird. In diesem Pufferbehälter kann sich eine organische Oberphase abscheiden. Die Verweilzeit dieser Oberphase, welche polymerisationsfähige Verbindungen enthält, in dem Pufferbehälter ist groß, in der Regel mehrere Tage. Damit steigt die Gefahr einer unerwünschten Polymerisation.

Zusätzlich zur Einstellung des pH-Wertes des der C₁-C₈-Alkoholrückgewinnung zugeführten Reaktionswassers können daher weitere Maßnahmen die Polymerisationsneigung in den wässrigen Phasen vermindern bzw. unterdrücken.

Diese zusätzlichen Maßnahmen umfassen, dass die Reaktionswasser führenden Leitungen und/oder Behälter
a) Kupfer enthalten oder
b) zumindest teilweise an der dem Reaktionswasser zugewandten Seite aus nichtmetallischen Werkstoffen, Kupfer oder Legierungen bestehen.

Die unter Punkt b) genannten Legierungen enthalten mindestens 10 Gew.-%, bevorzugt mindestens 12,5 Gew,-%, besonders bevorzugt mindestens 15 Gew.-% und insbesondere mindestens 16,5 Gew.-% Chrom sowie mindestens 5 Gew.-%, bevorzugt mindestens 7,5 Gew.-%, besonders bevorzugt mindestens 9 Gew.-% und insbesondere mindestens 10,5 Gew.-% Nickel.

Kupfer vermindert ebenfalls die unerwünschte Polymerisation im Reaktionswasser. Vorzugsweise wird elementares Kupfer verwendet. Kupfer kann in beliebiger Form in Reaktionswasser führende Anlagenteile wie beispielsweise Leitungen oder Behälter eingebracht werden, beispielsweise als Blech oder als Metallspäne, oder aber die Reaktionswasser führenden Anlagenteile werden zumindest teilweise aus Kupfer gefertigt.

Die Reaktionswasser führenden Anlagenteile wie beispielsweise Leitungen oder Behälter können aber auch zumindest teilweise aus Edelstahl gefertigt werden. Edelstähle sind Stähle mit einem Chromgehalt von mindestens 10 Gew.-%, bevorzugt mindestens 12,5 Gew,-%, besonders bevorzugt mindestens 15 Gew.-% und insbesondere mindestens 16,5 Gew.-% Chrom und einem Nickelgehalt von mindestens 5 Gew.-%, bevorzugt mindestens 7,5 Gew.-%, besonders bevorzugt mindestens 9 Gew.-% und insbesondere mindestens 10,5 Gew.-%. Die Verwendung nichtmetallischer Werkstoffe oder Auskleidungen, wie beispielsweise Polytetrafluorethylen oder glasfaserverstärkter Kunststoff, sind ebenfalls möglich.

Diese Maßnahmen stellen zusätzliche Maßnahmen dar, die neben der pH-Wert-Einstellung eine zusätzliche Möglichkeit zur Polymerisationsinhibierung in der wässrigen Phase der (Meth)acrylsäureesterherstellung bieten.

Im erfindungsgemäßen Verfahren werden C₁-C₈-Alkohole zur Veresterung der (Meth)acrylsäure eingesetzt. Dabei kommt üblicherweise jeder 1 bis 8 Kohlenstoffatome aufweisende Alkohol in Betracht, beispielsweise ein- oder mehrwertige Alkohole, bevorzugt ein- bis vierwertige Alkohole, besonders bevorzugt ein- bis dreiwertige, ganz besonders bevorzugt ein- oder zweiwertige und insbesondere einwertige.

Beispiele sind Methanol, Ethanol, n-Propanol, iso-Propanol, n-Butanol, sek.-Butanol, iso-Butanol, tert.-Butanol, Ethylenglykolmonomethylether, Ethylenglykolmonoethylether, Diethylenglykol, 1,3-Propandiolmonomethylether, 1,2-Propandiol, Ethylenglykol, 2,2-Dimethyl-1,3-propandiol, 1,3-Propandiol, 1,2-Butandiol, 1,4-Butandiol, Dimethylaminoethanol, n-Hexanol, n-Heptanol, n-Octanol, 2-Ethylhexanol, 3-Methylpentan-1,5-diol, 2-Ethylhexan-1,3-diol, 1,6-Hexandiol, Cyclopentanol, Cyclohexanol, Cyclooctanol, Triethylenglykol, Tetraethylenglykol, n-Pentanol, Trimethylolbutan, Trimethylolpropan, Trimethylolethan, Pentaerythrit, 2-Ethyl-1,3-propandiol, 2-Methyl-1,3-propandiol, 2-Ethyl-1,3-hexandiol, Glycerin.

Bevorzugte Alkohole sind Methanol, Ethanol, n-Butanol, iso-Butanol, sek.-Butanol, 2-Ethylhexanol, n-Octanol und Dimethylaminoethanol. Ganz besonders bevorzugt sind Methanol, Ethanol, n-Butanol, 2-Ethylhexanol und Dimethylaminoethanol.

Es können Gemische mehrerer Alkohole eingesetzt werden, beispielsweise 2 oder 3, bevorzugt wird jedoch nur ein Alkohol eingesetzt.

Die Herstellung von (Meth)acrylsäureestem erfolgt vielfältig auf an sich bekannte Weise durch Veresterung von (Meth)acrylsäure mit einem Alkohol, z. B. einem Alkanol. (Meth)acrylsäureester werden in der Regel über eine homogen oder heterogen katalysierte Veresterung erhalten, wie beispielsweise in Kirk Othmer, Encyclopedia of Chemical Technology, 4th Ed., 1994, Seiten 301-302 und Ullmann's Encyclopedia of Industrial Chemistry, 5. Auflage, Band A1, Seiten 167-169, beschrieben.

In der Literatur finden sich neben den im Stand der Technik genannten Verfahren zahlreiche Verfahren zur Herstellung von (Meth)acrylsäureestem durch Veresterung von (Meth)acrylsäure mit einem Alkohol, beispielsweise in den deutschen Offeniegungsschriften DE 196 04 252 A1 und DE 196 04 253 A1. Ein Verfahren zur Herstellung von Butylacrylat durch säurekatalysierte Veresterung von Acrylsäure mit Butanol wird z. B. in WO 98/52904 offenbart. Als Beispiel für eine diskontinuierliche säurekatalysierte Veresterung sei EP 0 890 568 A1 genannt.

Die verwendbaren sauren Katalysatoren sind bevorzugt Schwefelsäure, p-Toluolsulfonsäure, Benzolsulfonsäure, Dodecylbenzolsulfonsäure, Methansulfonsäure oder Gemische davon, denkbar sind auch saure Ionenaustauscher oder Zeolithe.

Besonders bevorzugt werden Schwefelsäure, p-Toluolsulfonsäure und Methansulfonsäure verwendet, ganz besonders bevorzugt sind Schwefelsäure und p-Toluolsulfonsäure.

Die Katalysatorkonzentration bezogen auf das Reaktionsgemisch beträgt beispielsweise 1 bis 20, bevorzugt 5 bis 15 Gew.-%.

Im erfindungsgemäßen Verfahren werden Stabilisatoren zur Polymerisationsinhibierung eingesetzt. Dabei eignen sich prinzipiell alle Polymerisationsinhibitoren, die zur Stabilisierung von (Meth)acrylsäure und (Meth)acrylsäureestem beispielsweise in DE 102 58 329 A1 empfohlen werden.

Geeignete Stabilisatoren können beispielsweise N-Oxide (Nitroxyl- oder N-Oxyl-Radikale, also Verbindungen, die wenigstens eine >N-O•-Gruppe aufweisen), wie z. B. 4-Hydroxy-2,2,6,6-tetramethylpiperidin-N-oxyl oder 4-Oxo-2,2,6,6-tetramethylpiperidin-N-oxyl, Phenole und Naphthole, wie p-Aminophenol, p-Nitrosophenol, 2-tert.-Butylphenol, 4-tert.-Butylphenol, 2,4-Di-tert.-butylphenol, 2-Methyl-4-tert.-butylphenol, 2,6-tert.-Butyl-4-methylphenol oder 4-tert.-Butyl-2,6-dimethylphenol, Chinone, wie z. B. Hydrochinon oder Hydrochinonmonomethylether, aromatische Amine, wie z. B. N,N-Diphenylamin, Phenylendiamine, wie z. B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z. B. N,N'-Dimethyl-p-phenylendiamin oder N,N'-Diethyl-p-phenylendiamin, Hydroxylamine, wie z.B. N,N-Diethylhydroxylamin, Imine, wie z. B. Methylethylimin oder Methylviolett, Sulfonamide, wie z. B. N-Methyl-4-toluolsulfonamid oder N-tert.-Butyl-4-toluolsulfonamid, Oxime, wie Aldoxime, Ketoxime oder Amidoxime, wie z. B. Diethylketoxim, Methylethylketoxim oder Salicylaldoxim, phosphorhaltige Verbindungen, wie z. B. Triphenylphosphit oder Triethylphosphit, schwefelhaltige Verbindungen, Metallsalze, wie z. B. Cer(III)acetat oder Cer(III)ethylhexanoat, oder Gemische davon sein.

Bevorzugt erfolgt die Stabilisierung mit Hydrochinon, Hydrochinonmonomethylether, 4-Hydroxy-2,2,6,6-tetramethylpiperidin-N-oxyl, 4-Oxo-2,2,6,6-tetramethylpiperidin-N-oxyl, 2,6-tert.-Butyl-4-methylphenol oder Gemischen davon.

Ganz besonders bevorzugt wird 4-Hydroxy-2,2,6,6-tetramethylpiperidin-N-oxyl als Polymerisationsinhibitor verwendet.

Das erfindungsgemäße Verfahren unterdrückt die Polymerisation im Reaktionswasser der (Meth)acrylsäureesterherstellung und erhöht damit die Anlagenverfügbarkeit. Es eignet sich somit für alle Herstellverfahren von (Meth)acrylsäureestern, die durch direkte Veresterung erhalten werden.

In dieser Schrift verwendete ppm- und Prozentangaben beziehen sich, falls nicht anders angegeben, auf Gewichtsprozente und -ppm.

### Beispiele

### Beispiel 1 bis 10

In Wasser wurden 0,5 Gew.-% Butylacrylat, 0,5 Gew.-% Acrylsäure und 100 Gew.-ppm Schwefeldioxid, jeweils bezogen auf die Menge an Wasser, gelöst. In den Beispielen 2,4,6,8 und 10 wurde der pH-Wert mit 5 Gew.-%iger Natronlauge auf pH 9 eingestellt. Jeweils 70 ml Lösung wurden mit verschiedenen Zusätzen in 100 ml Schraubdeckelflaschen bei 80 °C gelagert. Gemessen wurde die Zeit bis zur Trübung, d.h. bis zur sichtbaren Polymerisation.

| Beispiel | Zusatz | Zeit bis zur Trübung |
|---|---|---|
| | | [min] |
| 1 | Edelstahlblech¹ | 5 |
| 2 | Edelstahlblech¹, pH 9 | > 180 |
| 3 | Stahlblech² | 3 |
| 4 | Stahlblech², pH 9 | > 180 |
| 5 | Kupferblech | 65 |
| 6 | Kupferblech, pH 9 | > 180 |
| 7 | Eisendraht + Kupferblech | 7 |
| 8 | Eisendraht + Kupferblech, pH 9 | > 180 |
| 9 | ohne Zusatz | > 180 |
| 19 | ohne Zusatz, pH 9 | > 180 |

| | | |
|---|---|---|
| ¹ Edelstahlblech: Werkstoff 1.4571 gemäß DIN EN 10020, enthaltend 16,5-18,5 Gew.-% Chrom und 10,5-13,5 Gew.-% Nickel ² Stahlblech: Werkstoff 1.0426 gemäß DIN EN 10020, unlegierter Stahl | | |

## Patentansprüche

1. Verfahren zur Inhibierung der Polymerisation bei der Herstellung von (Meth)acrylsäureestem von C₁-C₈-Alkoholen, **dadurch gekennzeichnet, dass** der pH-Wert des der C₁-C₈-Alkoholrückgewinnung zugeführten Reaktionswassers größer 7 ist.

2. Verfahren nach Anspruch 1, **dadurch gekennzeichnet, dass** der pH-Wert ≥ 8 ist.

3. Verfahren nach Anspruch 1 oder 2, **dadurch gekennzeichnet, dass** der pH-Wert ≥ 9 ist.

4. Verfahren nach einem der Ansprüche 1 bis 3, **dadurch gekennzeichnet, dass** der pH-Wert mit einer alkalisch reagierenden Substanz ausgewählt aus Natriumhydroxid, Natriumcarbonat, Natriumhydrogencarbonat und deren Gemischen eingestellt wird.

5. Verfahren nach Anspruch 4, **dadurch gekennzeichnet, dass** zur Einstellung des pH-Wertes eine wässrige Lösung der alkalisch reagierenden Substanz eingesetzt wird.

6. Verfahren nach einem der Ansprüche 1 bis 5, **dadurch gekennzeichnet, dass** die Reaktionswasser führenden Leitungen und/oder Behälter
a) Kupfer enthalten oder
b) zumindest teilweise an der dem Reaktionswasser zugewandten Seite aus nichtmetallischen Werkstoffen, Kupfer oder Legierungen bestehen.

7. Verfahren nach Anspruch 6, **dadurch gekennzeichnet, dass** die Legierungen mindestens 10 Gew.-% Chrom und mindestens 5 Gew.-% Nickel enthalten.

8. Verfahren nach einem der Ansprüche 1 bis 7, **dadurch gekennzeichnet, dass** das Reaktionswasser über einen Pufferbehälter in die Rückgewinnung des C₁-C₈-Alkohols geführt wird.

## Claims

1. A process for inhibiting polymerization in the preparation of (meth)acrylic esters of C₁-C₈-alcohols, wherein the pH of the water of reaction supplied to the C₁-C₈-alcohol recovery is greater than 7.

2. The process according to claim 1, wherein the pH is ≥ 8.

3. The process according to claim 1 or 2, wherein the pH is ≥ 9.

4. The process according to any of claims 1 to 3, wherein the pH is adjusted with an alkaline substance selected from sodium hydroxide, sodium carbonate, sodium hydrogencarbonate and mixtures thereof.

5. The process according to claim 4, wherein the pH is adjusted using an aqueous solution of the alkaline substance.

6. The process according to any of claims 1 to 5, wherein the lines and/or vessels conducting the water of reaction
a) comprise copper or
b) at least partly at the side facing the water of reaction, consist of nonmetallic materials, copper or alloys.

7. The process according to claim 6, wherein the alloys comprise at least 10% by weight of chromium and at least 5% by weight of nickel.

8. The process according to any of claims 1 to 7, wherein the water or reaction is conducted via a buffer vessel into the recovery of the C₁-C₈-alcohol.

## Revendications

1. Procédé destiné à inhiber la polymérisation lors de la fabrication d'esters d'acide (méth)acrylique et d'alcools en C₁-C₈, **caractérisé en ce que** la valeur de pH de l'eau réactionnelle acheminée à la récupération de l'alcool en C₁-C₈ est supérieure à 7.

2. Procédé selon la revendication 1, **caractérisé en ce que** la valeur du pH est une valeur ≥ 8.

3. Procédé selon l'une quelconque des revendications 1 ou 2, **caractérisé en ce que** la valeur du pH est une valeur ≥ 9.

4. Procédé selon l'une quelconque des revendications 1 à 3, **caractérisé en ce que** la valeur du pH est ajustée avec une substance à réaction alcaline choisie parmi l'hydroxyde de sodium, le carbonate de sodium, l'hydrogéno carbonate de sodium et des mélanges de ceux-ci.

5. Procédé selon la revendication 4, **caractérisé en ce que** l'on utilise une solution aqueuse de la substance à réaction alcaline pour ajuster la valeur du pH.

6. Procédé selon l'une quelconque des revendications 1 à 5, **caractérisé en ce que** les conduits et/ou récipients acheminant l'eau réactionnelle :
a) contiennent du cuivre, ou
b) sont constitués au moins partiellement sur le côté orienté vers l'eau réactionnelle de matériaux non métalliques, de cuivre ou d'alliages.

7. Procédé selon la revendication 6, **caractérisé en ce que** les alliages contiennent au moins 10 % en poids de chrome et au moins 5 % en poids de nickel.

8. Procédé selon l'une quelconque des revendications 1 à 7, **caractérisé en ce que** l'eau réactionnelle est acheminée via un récipient de tampon à la récupération de l'alcool en C₁-C₈.
